# DEMANDE DE BREVET EUROPEEN

(11) **EP 1 944 302 A1**
(43) Date de publication de la demande: **16.07.2008**
(21) Numéro de dépôt: 08007270.5
(22) Date de dépôt: 17.06.2005
(51) Int. Cl.: C07D 333/38, A61K 31/381

(54) **Forme cristalline alpha du ranélate de strontium, son procédé de préparation et les compositions pharmaceutiques qui la contiennent**

(30) Priorité: 30.09.2004 FR 0410335
(62) Demande divisionnaire de: 05291297.9
(71) Demandeur: Les Laboratoires Servier, 92415 Courbevoie Cédex (FR)
(72) Inventeur: Horvath, Stéphane, 45380 La Chapelle-Saint-Mesmin (FR); Demuynck, Isabelle, 45000 Orleans (FR); Damien, Gérard, 45130 Meung-sur-Loire (FR)
(74) Mandataire: Giudicelli, Cathy

(57) **Abrégé**

Forme cristalline alpha du ranélate de strontium de formule (I): 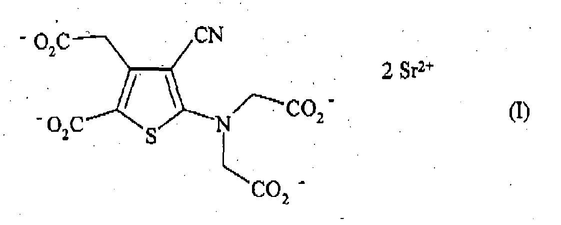 caractérisée par son diagramme de diffraction X sur poudre et par une teneur en eau comprise entre 22 et 24%;

Médicaments.

## Description

La présente invention concerne la forme cristalline alpha du ranélate de strontium, son procédé de préparation ainsi que les compositions pharmaceutiques qui la contiennent.

Le ranélate de strontium, représenté par la formule (I) : 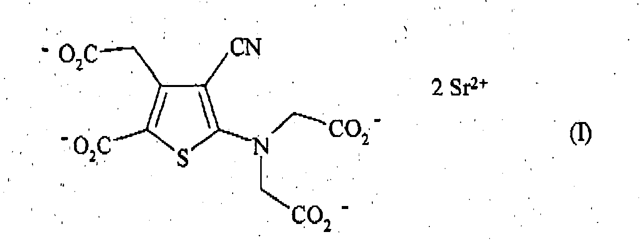 ou sel distrontique de l'acide 5-[bis(carboxyméthyl)amino]-3-carboxyméthyl-4-cyano-2-thiophènecarboxylique, ainsi que ses hydrates, possèdent des propriétés pharmacologiques et thérapeutiques très intéressantes, notamment des propriétés anti-ostéoporotiques remarquables, qui rendent ces composés utiles dans le traitement et la prévention des maladies osseuses.

Le ranélate de strontium, ainsi que ses hydrates, ont également des propriétés qui les rendent utiles dans le traitement et la prévention de l'arthrose.

La préparation et l'utilisation en thérapeutique du ranélate de strontium et de ses tétrahydrate, heptahydrate et octahydrate ont été décrits dans le brevet européen EP 0415 850.

L'utilisation du ranélate de stronium dans la prévention et le traitement de l'arthrose a été décrite dans le brevet européen EP 0 813 869.

La demanderesse a présentement trouvé que le ranélate de strontium pouvait être obtenu sous une forme cristalline bien définie, parfaitement reproductible et présentant de ce fait des caractéristiques intéressantes de filtration et de facilité de formulation.

Plus spécifiquement, la présente invention concerne la forme cristalline alpha du ranélate de strontium, caractérisée par une teneur en eau comprise entre 22 et 24% et par le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å):

| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 7,6 | 4527 | 448 | 0,1004 | 11,649 |
| 2 | 8,0 | 1438 | 142 | 0,1004 | 11,069 |
| 3 | 8,3 | 3522 | 349 | 0,1004 | 10,642 |
| 4 | 8,6 | 11347 | 1123 | 0,1004 | 10,272 |
| 5 | 8,9 | 7332 | 726 | 0,1004 | 9,889 |
| 6 | 11,0 | 1047 | 104 | 0,1004 | 8,072 |
| 7 | 11,3 | 1655 | 164 | 0,1004 | 7,840 |
| 8 | 12,0 | 2186 | 216 | 0,1004 | 7,355 |
| 9 | 13,2 | 2887 | 381 | 0,1338 | 6,703 |
| 10 | 13,5 | 1705 | 169 | 0,1004 | 6,557 |
| 11 | 14,1 | 154 | 30 | 0,2007 | 6,275 |
| 12 | 14,7 | 803 | 79 | 0,1004 | 6,035 |
| 13 | 14,9 | 1346 | 178 | 0,1338 | 5,942 |
| 14 | 15,8 | 1556 | 154 | 0.1004 | 5,613 |
| 15 | 16.0 | 3339 | 441 | 0,1338 | 5,527 |
| 16 | 16,7 | 1845 | 183 | 0,1004 | 5,308 |
| 17 | 17,3 | 2835 | 281 | 0,1004 | 5,127 |
| 18 | 17,6 | 1252 | 124 | 0,1004 | 5,049 |
| 19 | 18,0 | 2183 | 216 | 0,1004 | 4,939 |
| 20 | 19,2 | 2303 | 228 | 0,1004 | 4,622 |
| 21 | 19,8 | 1298 | 128 | 0,1004 | 4,475 |
| 22 | 20,3 | 788 | 78 | 0,1004 | 4,373 |
| 23 | 20,6 | 1039 | 103 | 0,1004 | 4,317 |
| 24 | 21,1 | 882 | 116 | 0,1338 | 4,211 |
| 25 | 21,7 | 390 | 38 | 0,1004 | 4,103 |
| 26 | 22,3 | 1919 | 253 | 0,1338 | 3,990 |
| 27 | 22,7 | 1805 | 179 | 0,1004 | 3,923 |
| 28 | 23,0 | 4043 | 467 | 0,1171 | 3,861 |
| 29 | 23,5 | 650 | 86 | 0,1338 | 3,792 |
| 30 | 24,0 | 8677 | 1002 | 0,1171 | 3,711 |
| 31 | 24,7 | 229 | 30 | 0,1338 | 3,600 |
| 32 | 25,1 | 1246 | 164 | 0,1338 | 3,543 |
| 33 | 25,6 | 1659 | 219 | 0,1338 | 3,473 |
| 34 | 25,9 | 1773 | 175 | 0,1004 | 3,442 |
| 35 | 26,3 | 695 | 69 | 0,1004 | 3,385 |
| 36 | 26,6 | 401 | 46 | 0,1171 | 3,355 |
| 37 | 27,0 | 2800 | 370 | 0,1338 | 3,300 |
| 38 | 27,6 | 1415 | 140 | 0,1004 | 3,230 |
| 39 | 28,0 | 3250 | 429 | 0,1338 | 3,186 |
| 40 | 28,4 | 1513 | 250 | 0.1673 | 3,144 |
| 41 | 29,1 | 1456 | 144 | 0,1004 | 3,068 |
| 42 | 29,6 | 1943 | 192 | 0,1004 | 3,022 |
| 43 | 30,1 | 3637 | 540 | 0,1506 | 2,967 |
| 44 | 30,5 | 707 | 117 | 0,1673 | 2,929 |
| 45 | 30,9 | 596 | 59 | 0,1004 | 2,897 |
| 46 | 31,8 | 577 | 76 | 0,1338 | 2,816 |
| 47 | 32,0 | 1080 | 107 | 0,1004 | 2,796 |
| 48 | 32,5 | 512 | 51 | 0,1004 | 2,756 |
| 49 | 32,9 | 1268 | 167 | 0,1338 | 2,726 |
| 50 | 33,4 | 1180 | 117 | 0,1004 | 2,685 |

L'invention s'étend également à un procédé de préparation de la forme cristalline alpha du ranélate de strontium, caractérisé en ce que l'on porte à reflux une solution du ranélate de strontium ou de l'un de ses hydrates dans l'eau, puis on refroidit jusqu'à cristallisation complète et on recueille le produit par filtration.
- Dans le procédé de préparation selon l'invention, on peut utiliser le ranélate de strontium, ou l'un de ses hydrates, obtenu par n'importe quel procédé, par exemple l'octahydrate du ranélate de strontium obtenu par le procédé de préparation décrit dans le brevet EP 0415 5 850.
- L'obtention de cette forme cristalline a pour avantage de permettre une filtration particulièrement rapide et efficace, ainsi que la préparation de formulations pharmaceutiques ayant une composition constante et reproductible, ce qui est particulièrement avantageux lorsque ces formulations sont destinées à l'administration orale.
- La forme ainsi obtenue est suffisamment stable pour autoriser son stockage prolongé sans conditions particulières de température, de lumière, d'humidité ou de taux d'oxygène.

L'invention s'étend aussi aux compositions pharmaceutiques renfermant comme principe actif la forme cristalline alpha du ranélate de strontium avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 0,2 g à 10 g par jour en une ou plusieurs prises.

Les exemples suivants illustrent l'invention.

Le spectre de diffraction X sur poudre a été mesuré avec les conditions expérimentales suivantes :
- Diffractomètre PANalytical X'Pert Pro, détecteur X'Celerator,
- Tension 45 KV, intensité 40mA,
- Montage θ-θ,
- Filtre Kβ (Ni),
- fente de soller sur le faisceau incident et sur le faisceau diffracté : 0,04 rad,
- fentes de divergence: automatique, longueur irradiée : 10 mm,
- Masque : 10 mm,
- Fente anti-diffusion : 1/2°,
- Mode de mesure: continu de 3° à 34°, avec une incrémentation de 0,017°,
- Temps de mesure par pas : 31,1 s,
- Temps total: 8 min 07 s,
- Vitesse de mesure : 0,068 °/s,
- Spinner : révolution de 1 tour/s,
- Température de mesure : ambiante.

### EXEMPLE 1 : Forme cristalline alpha du ranélate de strontium

200 g de l'octahydrate du ranélate de strontium obtenu selon le procédé décrit dans le brevet EP 0415 850 sont mélangés à 21 d'eau et portés au reflux.
Le milieu est ensuite refroidi jusqu'à 20°C.
Le solide obtenu est collecté par filtration.

La teneur en eau du produit obtenu, déterminée par perte à la dessiccation, est comprise entre 22 et 24%, ce qui correspond à un nombre de molécules d'eau compris entre 8,1 et 9 par molécule de ranélate de strontium

### Diagramme de diffraction X sur poudre :

Le profil de diffraction des rayons X de la poudre (angles de diffraction) de la forme alpha du ranélate de strontium est donné par les raies significatives rassemblées dans le tableau suivant :

| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 7,6 | 4527 | 448 | 0.1004 | 11,649 |
| 2 | 8,0 | 1438 | 142 | 0,1004 | 11,069 |
| 3 | 8,3 | 3522 | 349 | 0,1004 | 10,642 |
| 4 | 8,6 | 11347 | 1123 | 0,1004 | 10,272 |
| 5 | 8.9 | 7332 | 726 | 0,1004 | 9,889 |
| 6 | 11,0 | 1047 | 104 | 0,1004 | 8,072 |
| 7 | 11,3 | 1655 | 164 | 0,1004 | 7,840 |
| 8 | 12,0 | 2186 | 216 | 0,1004 | 7,355 |
| 9 | 13,2 | 2887 | 381 | 0,1338 | 6,703 |
| 10 | 13,5 | 1705 | 169 | 0,1004 | 6,557 |
| 11 | 14,1 | 154 | 30 | 0,2007 | 6,275 |
| 12 | 14,7 | 803 | 79 | 0,1004 | 6,035 |
| 13 | 14,9 | 1346 | 178 | 0,1338 | 5,942 |
| 14 | 15,8 | 1556 | 154 | 0,1004 | 5,613 |
| 15 | 16,0 | 3339 | 441 | 0,1338 | 5,527 |
| 16 | 16,7 | 1845 | 183 | 0,1004 | 5,308 |
| 17 | 17,3 | 2835 | 281 | 0,1004 | 5,127 |
| 18 | 17,6 | 1252 | 124 | 0,1004 | 5.049 |
| 19 | 18,0 | 2183 | 216 | 0,1004 | 4,939 |
| 20 | 19,2 | 2303 | 228 | 0,1004 | 4,622 |
| 21 | 19,8 | 1298 | 128 | 0,1004 | 4,475 |
| 22 | 20,3 | 788 | 78 | 0,1004 | 4,373 |
| 23 | 20,6 | 1039 | 103 | 0,1004 | 4,317 |
| 24 | 21,1 | 882 | 118 | 0,1338 | 4,211 |
| 25 | 21,7 | 390 | 38 | 0,1004 | 4,103 |
| 26 | 22,3 | 1919 | 253 | 0,1338 | 3,990 |
| 27 | 22,7 | 1805 | 179 | 0,1004 | 3,923 |
| 28 | 23,0 | 4043 | 467 | 0,1171 | 3,861 |
| 29 | 23,5 | 650 | 86 | 0,1338 | 3,792 |
| 30 | 24,0 | 8677 | 1002 | 0,1171 | 3,711 |
| 31 | 24,7 | 229 | 30 | 0,1338 | 3,600 |
| 32 | 25,1 | 1246 | 164 | 0,1338 | 3,543 |
| 33 | 25,6 | 1659 | 219 | 0,1338 | 3,473 |
| 34 | 25,9 | 1773 | 175 | 0,1004 | 3,442 |
| 35 | 26,3 | 695 | 69 | 0,1004 | 3,385 |
| 36 | 26,6 | 401 | 46 | 0,1171 | 3,355 |
| 37 | 27,0 | 2800 | 370 | 0,1338 | 3,300 |
| 38 | 27,6 | 1415 | 140 | 0,1004 | 3,230 |
| 39 | 28,0. | 3250 | 429 | 0,1338 | 3,166 |
| 40 | 28,4 | 1513 | 250 | 0,1673 | 3,144 |
| 41 | 29,1 | 1456 | 144 | 0,1004 | 3,068 |
| 42 | 29,6 | 1943 | 192 | 0,1004 | 3,022 |
| 43 | 30,1 | 3837 | 540 | 0,1506 | 2,967 |
| 44 | 30,5 | 707 | 117 | 0,1673 | 2,929 |
| 45 | 30,9 | 596 | 59 | 0,1004 | 2,897 |
| 46 | 31,8 | 577 | 76 | 0,1338 | 2,816 |
| 47 | 32,0 | 1080 | 107 | 0,1004 | 2,796 |
| 48 | 32,5 | 512 | 51 | 0,1004 | 2,756 |
| 49 | 32,9 | 1268 | 167 | 0.1338 | 2,726 |
| 50 | 33,4 | 1160 | 117 | 0,1004 | 2,685 |

### EXEMPLE 2 : Composition pharmaceutique

Formule de préparation pour 1000 comprimés dosés à 0,5 g :

| | |
|---|---|
| Composé de l'exemple 1 | 658 g |
| Carboxyméthylamidon sodique | 25,5 g |
| Cellulose microcristalline 119,4 | g |
| Povidone | 38 g |
| Silice colloïdale anhydre 1,5 | g |
| Stéarate de magnésium | 7,6 g |

## Revendications

1. Forme cristalline alpha du ranélate de strontium de formule (I) : 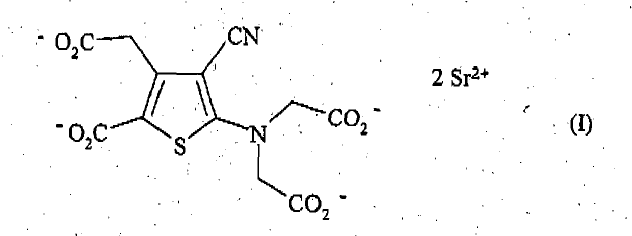 **caractérisée par** une teneur en eau comprise entre 22 et 24%, et par le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å) :
| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 7,6 | 4527 | 448 | 0,1004 | 11,649 |
| 2 | 8,0 | 1438 | 142 | 0,1004 | 11,069 |
| 3 | 8,3 | 3522 | 349 | 0,1004 | 10,642 |
| 4 | 8,6 | 11347 | 1123 | 0,1004 | 10,272 |
| 5 | 8,9 | 7332 | 726 | 0,1004 | 9,889 |
| 6 | 11,0 | 1047 | 104 | 0,1004 | 8,072 |
| 7 | 11,3 | 1655 | 164 | 0,1004 | 7,840 |
| 8 | 12,0 | 2186 | 216, | 0,1004 | 7,355 |
| 9 | 13,2 | 2887 | 381 | 0,1338 | 6,703 |
| 10 | 13,5 | 1705 | 169 | 0,1004 | 6,557 |
| 11 | 14,1 | 154 | 30 | 0,2007 | 6,275 |
| 12 | 14,7 | 803 | 79 | 0,1004 | 6,035 |
| 13 | 14,9 | 1346 | 178 | 0,1338 | 5,942 |
| 14 | 15,8 | 1556 | 154 | 0,1004 | 5,613 |
| 15 | 16,0 | 3339 | 441 | 0,1338 | 6,527 |
| 16 | 16,7 | 1845 | 183 | 0,1004 | 5,308 |
| 17 | 17,3 | 2835 | 281 | 0,1004 | 5,127 |
| 18 | 17,6 | 1252 | 124 | 0,1004 | 5,049 |
| 19 | 18,0 | 2183 | 216 | 0.1004 | 4,939 |
| 20 | 19,2 | 2303 | 228 | 0,1004 | 4,622 |
| 21 | 19,8 | 1298 | 128 | 0,1004 | 4,475 |
| 22 | 20,3 | 788 | 78 | 0,1004 | 4,373 |
| 23 | 20,6 | 1039 | 103 | 0,1004 | 4,317 |
| 24 | 21,1 | 882 | 116 | 0,1338 | 4,211 |
| 25 | 21,7 | 390 | 38 | 0,1004 | 4,103 |
| 26 | 22,3 | 1919 | 253 | 0,1338 | 3,990 |
| 27 | 22,7 | 1805 | 179 | 0,1004 | 3,923 |
| 28 | 23,0 | 4043 | 467 | 0,1171 | 3,861 |
| 29 | 23,5 | 650 | 86 | 0,1338 | 3,792 |
| 30 | 24,0 | 8677 | 1002 | 0,1171 | 3,711 |
| 31 | 24,7 | 229 | 30 | 0,1338 | 3,600 |
| 32 | 25,1 | 1246 | 164 | 0,1338 | 3,543 |
| 33 | 25,6 | 1659 | 219 | 0,1338 | 3,473 |
| 34 | 25,9 | 1773 | 175 | 0,1004 | 3,442 |
| 35 | 26,3 | 695 | 69 | 0,1004 | 3,385 |
| 36 | 26,6 | 401 | 46 | 0,1171 | 3,355 |
| 37 | 27,0 | 2800 | 370 | 0,1338 | 3,300 |
| 38 | 27,6 | 1415 | 140 | 0,1004 | 3,230 |
| 39 | 28,0 | 3250 | 429 | 0,1338 | 3,186 |
| 40 | 28,4 | 1513 | 250 | 0,1673 | 3,144 |
| 41 | 29,1 | 1456 | 144 | 0,1004 | 3,068 |
| 42 | 29,6 | 1943 | 192 | 0,1004 | 3,022 |
| 43 | 30,1 | 3637 | 540 | 0,1506 | 2,967 |
| 44 | 30,5 | 707 | 117 | 0,1673 | 2,929 |
| 45 | 30,9 | 596 | 59 | 0,1004 | 2,897 |
| 46 | 31.8 | 577 | 76 | 0,1338 | 2.816 |
| 47 | 32,0 | 1080 | 107 | 0,1004 | 2,796 |
| 48 | 32,5 | 512 | 51 | 0,1004 | 2,756 |
| 49 | 32,9 | 1268 | 167 | 0,1338 | 2,726 |
| 50 | 33,4 | 1180 | 117 | 0,1004 | 2,685 |

2. Procédé de préparation de la forme cristalline alpha du ranélate de strontium selon la revendication 1, **caractérisé en ce que** l'on porte à reflux une solution du ranélate de strontium ou de l'un de ses hydrates dans l'eau, puis on refroidit jusqu'à cristallisation complète et on recueille le produit par filtration.

3. Composition pharmaceutique contenant comme principe actif la forme cristalline alpha du ranélate de strontium selon la revendication 1, en combinaison avec un ou plusieurs véhicules inertes, non toxiques et pharmaceutiquement acceptables.

4. Utilisation de la forme cristalline alpha du ranélate de strontium selon la revendication 1, pour la fabrication de médicaments utiles dans le traitement ou la prévention de l'ostéoporose.

5. Utilisation de la forme cristalline alpha du ranélate de strontium selon la revendication 1, pour la fabrication de médicaments utiles dans le traitement ou la prévention de l'arthrose.
